# EUROPEAN PATENT APPLICATION

(11) **EP 3 128 452 A1**
(43) Date of publication of application: **08.02.2017**
(21) Application number: 14887847.3
(22) Date of filing: 24.10.2014
(51) Int. Cl.: G06F 21/32, A61B 5/117, G06T 7/00

(54) **SERVER, NETWORK SYSTEM, AND PERSONAL AUTHENTICATION METHOD**

(30) Priority: 31.03.2014 JP 2014073539
(71) Applicant: Fujitsu Frontech Limited, Inagi-shi, Tokyo 206-8555 (JP)
(72) Inventor: IWAGUCHI, Isao, Inagi-shi Tokyo 206-8555 (JP); YAMAZAKI, Kozo, Inagi-shi Tokyo 206-8555 (JP)
(74) Representative: Hutchison, James
(86) International application number: PCT/JP2014/078397
(87) International publication number: WO 2015/151324

(57) **Abstract**

A server that enables personal authentication even when noise components included in a registration template that is registered in advance and noise components included in a collation template that is acquirable at the time of personal authentication differ from each other. In this server, a template pair DB (11) includes template pairs each of which includes a UP template and an LP template that are acquired from the same person, for multiple persons. A template collating unit (12) collates a UP template that is transmitted from a mobile terminal with the template pairs for the multiple persons, while collating an LP that is transmitted from a transaction server with the template pairs of the multiple persons, and performs personal authentication according to whether a template pair including a UP template that coincides with the UP template transmitted from the mobile terminal and a template pair including an LP template that coincides with the LP template transmitted from the transaction server are an identical template pair.

## Description

### Technical Field

The present invention relates to a server, a network system, and a personal authentication method.

### Background Art

As one of techniques of performing personal authentication using biometric information, there has been a technique of performing personal authentication by using palm vein patterns. Moreover, as one of the techniques of performing personal authentication by using pal vein patterns, there has been "noncontact vein authentication". In this noncontact vein authentication, a vein pattern is acquired by imaging the inside of a palm while shining light on the palm.

As for devices to perform noncontact vein authentication, various devices applying various acquisition methods for acquiring vein patterns have been available. For example, as a first acquisition method, there is one acquiring a vein pattern by applying linearly polarized light from illumination to a palm, and by extracting components that are perpendicular to the polarization direction of the illumination out of light passed through the palm. In the following, this first acquisition method is referred to as "linear polarization method" in some cases. In the linear polarization method, because surface reflected light of a palm is removed, a clearer vain pattern can be acquired. Furthermore, for example, as a second acquisition method, there is one acquiring a vein pattern by using unpolarized diffused light. In the following, this second acquisition method is referred to as "unpolarization method" in some cases. In the unpolarization method, by using diffused light, light change noise that is generated by a change in brightness due to unevenness on a skin surface of a palm can be suppressed. Moreover, a device of the unpolarization method generally has a simple device structure compared to a device of the linear polarization method.

Moreover, as a device that can acquire an image by the linear polarization and an image by the unpolarization method at the same time, there is one described in Patent Literature 1.

### Citation List

### Patent Literature

Patent Literature 1: Japanese Patent No. 4974543

### Summary of invention

### Technical Problem

By extracting characteristics of a vein pattern from an acquired vein pattern, a "template" is created. By collating a "registered template" that is created in advance and registered in a database and a "collation template" that is created at the time of authentication, personal authentication is performed. In a template, components of light change noise (hereinafter, simply referred to as "noise components" in some cases) that is generated by a change in brightness due to unevenness of a skin surface are included. The noise components included in a template differ depending on an acquisition method of a vein pattern. For example, noise components included in a template created from a vein pattern that is acquired by the linear polarization method (hereinafter, referred to as "linear polarization template" in some cases) and noise components included in a template created from a vein pattern that is acquired by the unpolarization method (hereinafter, referred to as "unpolarization template" in some cases) differ from each other. Therefore, when vein patterns of the same person are acquired by the linear polarization method and by the unpolarization method at the same time, the linear polarization template and an unpolarization template are not compatible with each other. Therefore, conventionally, even when a linear polarization template has been available as a registration template, if the collation template is an unpolarization template, personal authentication cannot be performed.

The disclosed technique has been achieved in view of the above problem, and an object thereof is to provide a server, a network system, and a personal authentication method that enable personal authentication even when noise components included in a registered template and noise components included in a collation template differ from each other.

### Solution to Problem

In one aspect of the disclosed embodiment, a server includes a database that stores template pairs for a plurality of persons, the template pairs each pairing a first template and a second template that are acquired by using a first polarization method and a second polarization method different from each other, respectively, from a same person, and a collating unit that collates a third template by the first polarization method, the third template being transmitted from a terminal device, with the template pairs for the persons, collates a fourth template by the second polarization method, the fourth template being transmitted from another server, with the template pairs for the persons, and performs personal authentication according to whether a first template pair and a second template pair are an identical template pair, the first template pair including the first template that coincides with the third template, and the second template pair including the second template that coincides with the fourth template.

### Advantageous Effects of Invention

According to disclosed embodiments, personal authentication is possible even when noise components included in a registered template and noise components included in a collation template differ from each other.

### Brief Description of Drawings

FIG. 1 is a diagram depicting one example of a configuration of a network system of a first embodiment.
FIG. 2 is a functional block diagram depicting one example of an intermediate server of the first embodiment.
FIG. 3 is a functional block diagram depicting one example of a transaction server of the first embodiment.
FIG. 4 is a functional block diagram depicting one example of a mobile terminal of the first embodiment.
FIG. 5 is diagram for explaining relation between templates of the first embodiment.
FIG. 6 is a diagram depicting one example of a processing sequence of a network system of the first embodiment.
FIG. 7 is a functional block diagram depicting one example of an intermediate server of a second embodiment.
FIG. 8 is a functional block diagram depicting one example of a transaction server of the second embodiment.
FIG. 9 is a diagram depicting one example of a processing sequence of a network system of the second embodiment.
FIG. 10 is a diagram depicting one example of a configuration of a network system of a third embodiment.
FIG. 11 is a functional block diagram depicting one example of a transaction server of the third embodiment.
FIG. 12 is a functional block diagram depicting one example of a fixed terminal of the third embodiment.
FIG. 13 is a diagram depicting one example of a processing sequence of a network system of the third embodiment.
FIG. 14 is a functional block diagram depicting one example of an intermediate server of a fourth embodiment.
FIG. 15 is a functional block diagram depicting one example of a transaction server of the fourth embodiment.
FIG. 16 is a diagram depicting one example of a processing sequence of a network system of the fourth embodiment.
FIG. 17 is a functional block diagram depicting one example of an intermediate server of a fifth embodiment.
FIG. 18 is a functional block diagram depicting one example of a transaction server of the fifth embodiment.
FIG. 19 is a diagram depicting one example of a processing sequence of a network system of the fifth embodiment.
FIG. 20 is a functional block diagram depicting one example of a transaction server of a sixth embodiment.
FIG. 21 is a diagram depicting one example of a processing sequence of a network system of a sixth embodiment.
FIG. 22 is a diagram depicting a hardware configuration example of an intermediate server, a transaction server, a mobile terminal, and a fixed terminal.

### Embodiments for Carrying Out the Invention

Embodiments of a server, a network system, and a personal authentication method disclosed in the present application are explained in detail below based on the drawings. The server, the network system, and the personal authentication method disclosed in the present application are not limited to the embodiments. In addition, an identical reference symbol is assigned to components having an identical function, and steps performing identical processing in each embodiment, and duplicated explanation thereof is omitted.

### [First Embodiment]

### <Configuration of Network System>

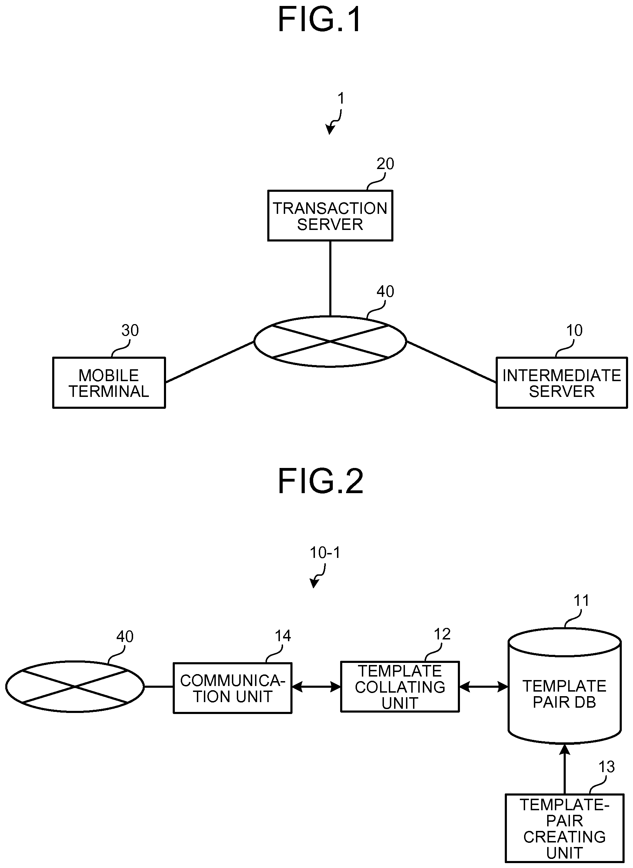

FIG. 1 is a diagram depicting one example of a configuration of a network system of a first embodiment. A network system 1 depicted in FIG. 1 includes an intermediate server 10, a transaction server 20, and a mobile terminal 30. The intermediate server 10, the transaction server 20, and the mobile terminal 30 are connected through a network 40.

### <Configuration of Intermediate Server>

FIG. 2 is a functional block diagram depicting one example of the intermediate server of the first embodiment. An intermediate server 10-1 depicted in FIG. 2 corresponds to the intermediate server 10 depicted in FIG. 1. The intermediate server 10-1 includes a template pair DB (database) 11, a template collating unit 12, a template-pair creating unit 13, and a communication unit 14 that is connected to the network 40.

In the template pair DB 11, a linear polarization template and an unpolarization template that are acquired at the same time from the same person are paired, and stored as a template pair. For example, in the template pair DB 11, template pairs of 1000 people each constituted of a linear polarization template and an unpolarization template are stored with template IDs (identifiers) "C000" to "C999" added thereto, and each template pair can be uniquely identified by a template ID. For example, for a person "A", a template pair of ID=C897 is stored, and for a person "B", a template pair of ID=C523 is stored. In the following, the linear polarization template is referred to as "LP (linear polarization) template", and the unpolarization template is referred to as "UP (unpolarization) template" in some cases.

The template-pair creating unit 13 uses, for example, the technique disclosed in the specification of Japanese Patent No. 4974543, and thereby acquires a vein pattern by the linear polarization method and a vein pattern by the unpolarization method at a time by a single time of imaging from the same person, and creates an LP template and a UP template from the vein patterns that are acquired at a time from the same person. The template-pair creating unit 13 pairs an LP template and a UP template that are created from multiple vein patterns acquired at a time from the same person to create a template pair of each person, and outputs the created template pair to the template pair DB 11.

The template collating unit 12 collates a collation template that is input from the communication unit 14 and a template pair that is stored in the template pair DB 11, and outputs an authentication result to the communication unit 14.

The communication unit 14 outputs an authentication request that is received from the mobile terminal 30 to the template collating unit 12. Moreover, the communication unit 14 outputs a collation template that is received from the transaction server 20 or the mobile terminal 30 to the template collating unit 12. Furthermore, the communication unit 14 transmits an authentication result that is input from the template collating unit 12 to the transaction server 20 or the mobile terminal 30.

### <Configuration of Transaction Server>

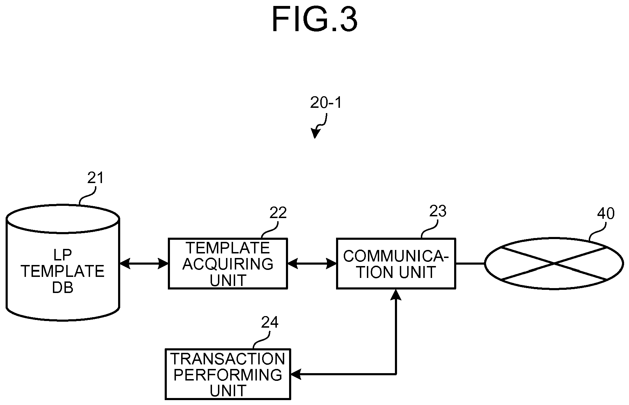

FIG. 3 is a functional block diagram depicting one example of a transaction server of the first embodiment. A transaction server 20-1 depicted in FIG. 3 corresponds to the transaction server 20 depicted in FIG. 1. The transaction server 20-1 includes an LP template DB (database) 21, a template acquiring unit 22, a communication unit 23 that is connected to the network 40, and a transaction performing unit 24.

In the LP template DB 21, multiple LP templates that are respectively acquired from multiple persons are stored as registration templates. For example, in the LP template DB 21, LP templates of 1000 people are stored with template IDs "A000" to "A999" added thereto, and each LP template can be uniquely identified by a template ID. For example, for the person "A", an LP template of ID=A123 is stored, and for the person "B", an LP template of ID=A567 is stored.

The template acquiring unit 22 acquires an LP template from the LP template DB 21 in response to a template transmission request that is input from the communication unit 23, and outputs it to the communication unit 23.

The communication unit 23 transmits an LP template that is input from the template acquiring unit 22 to the intermediate server 10-1. Moreover, the communication unit 14 outputs an authentication result that is received from the intermediate server 10-1 to the transaction performing unit 24. Furthermore, the communication unit 23 receives, from the mobile terminal 30, a command (hereinafter, referred to as "transaction command" in some cases) that is communicated between the mobile terminal 30 and the transaction server 20 for a transaction performed between the mobile terminal 30 and the transaction server 20, and outputs it to the transaction performing unit 24. Moreover, the communication unit 23 transmits a transaction command that is input from the transaction performing unit 24 to the mobile terminal 30.

The transaction performing unit 24 performs a transaction between itself and the mobile terminal 30 through the network 40 according to an authentication result input from the communication unit 23. The transaction performing unit 24 performs a transaction between itself and the mobile terminal 30 through the network 40 by receiving various kinds of transaction commands input from the communication unit 23, and by outputting various kinds of transaction commands to the communication unit 23.

### <Configuration of Mobile Terminal>

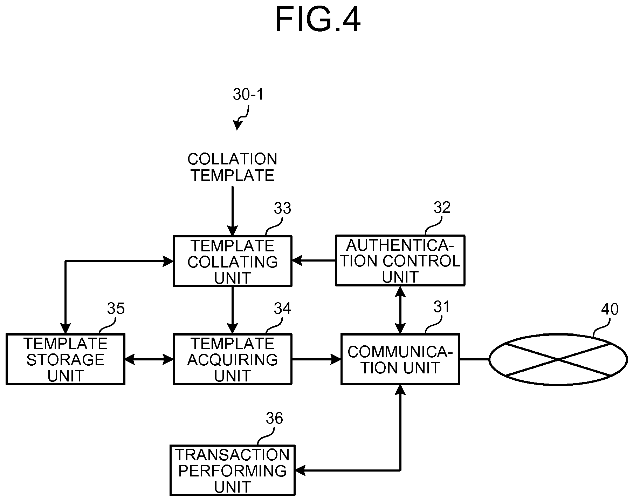

FIG. 4 is a functional block diagram depicting one example of a mobile terminal of the first embodiment. A mobile terminal 30-1 depicted in FIG. 4 corresponds to the mobile terminal 30 depicted in FIG. 1. The mobile terminal 30-1 includes a communication unit 31 that is connected to the network 40, an authentication control unit 32, a template collating unit 33, a template acquiring unit 34, a template storage unit 35, and a transaction performing unit 36.

In the template storage unit 35, a UP template that is registered in advance by a user of the mobile terminal 30-1 is stored as a registration template. For example, in the template storage unit 35, a UP template of the user of the mobile terminal 30-1 is stored with a unique user ID "B432" added thereto. For example, the user of the mobile terminal 30-1 is the above person "A". That is, the user ID of the person "A" is "B432".

The template collating unit 33 collates a UP template that is input as a collation template, and a UP template that is stored as a registration template in the template storage unit 35, and outputs an authentication result to the template acquiring unit 34. The UP template input to the template collating unit 33 is one acquired from a vein pattern that is imaged by the unpolarization method by an imaging unit (not depicted) included in the mobile terminal 30-1. A collation-template creating unit (not depicted) included in the mobile terminal 30-1 creates a UP template as a collation template from a vein pattern that is imaged by the unpolarization method, and outputs it to the template collating unit 33.

The template acquiring unit 34 acquires a UP template from the template storage unit 35 according to an authentication result input from the template collating unit 33, and outputs it to the communication unit 31.

The authentication control unit 32 inputs and outputs various kinds of control signals to perform personal authentication using the mobile terminal 30-1 to and from the communication unit 31, and outputs to the template collating unit 33.

The communication unit 31 transmits a UP template input from the template acquiring unit 34 to the intermediate server 10-1. Moreover, the communication unit 31 outputs a control signal received from the intermediate server 10-1 or the transaction server 20-1 to the authentication control unit 32. Furthermore, the communication unit 31 outputs an authentication result received from the intermediate server 10-1 to the transaction performing unit 36. Moreover, the communication unit 31 receives a transaction command from the transaction server 20-1, to output to the transaction performing unit 36. Furthermore, the communication unit 31 transmits a transaction command input from the transaction performing unit 36, to the transaction server 20-1.

The transaction performing unit 36 performs a transaction between itself and the transaction server 20-1 through the network 40 according to an authentication result input from the communication unit 31. The transaction performing unit 36 performs a transaction between itself and the transaction server 20-1 through the network 40 by receiving various kinds of transaction commands input from the communication unit 31, and by outputting various kinds of transaction commands to the communication unit 31.

### <Relation Between Templates>

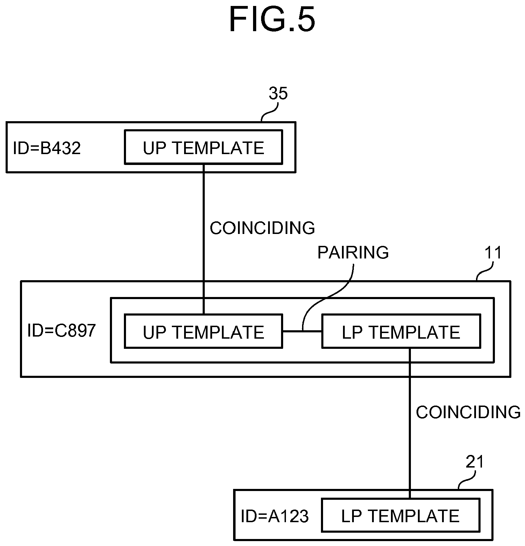

FIG. 5 is diagram for explaining relation between templates of the first embodiment.

As described above, in the template pair DB 11 of the intermediate server 10-1, the template pair of ID=C897 is stored for the person "A". That is, in the template pair DB 11 of the intermediate server 10-1, a UP template and an LP template that are acquired at the same time from the same person "A" are paired, and ID=C897 is assigned to this template pair. On the other hand, in the template storage unit 35 of the mobile terminal 30-1, the UP template acquired from the person "A" is stored with the user ID=B432 of the person "A" assigned thereto. Moreover, in the LP template DB 21 of the transaction server 20-1, the LP template of ID=A123 is stored for the person "A".

Because the mobile terminal 30-1 only supports the unpolarization method, while a UP template can be acquired, an LP template cannot be acquired. On the other hand, in the transaction server 20-1, although an LP template is stored, a UP template is not stored. In addition, as described above, because an LP template and a UP template are not compatible with each other, personal authentication from the mobile terminal 30-1 to the transaction server 20-1 cannot be performed by collating the UP template acquired in the mobile terminal 30-1 and the LP template stored in the transaction server 20-1. That is, personal authentication for the transaction server 20-1 cannot be performed directly from the mobile terminal 30-1.

On the other hand, the UP template of ID=B432 that is stored in the template storage unit 35 of the mobile terminal 30-1 and the UP template included in the template pair of ID=897 that is stored in the template pair DB 11 of the intermediate server 10-1 are both acquired from the person "A", and therefore, the UP templates of the both coincide with each other. Moreover, the LP template of ID=A123 that is stored in the LP template DB 21 of the transaction server 20-1 and the LP template included in the template pair of ID=C897 that is stored in the template pair DB 11 of the intermediate server 10-1 are both acquired from the person "A", and therefore, the LP templates of the both coincide with each other. Furthermore, the UP template and the LP template included in the template pair of ID=C897 are paired. Note that "template of the both coincide with each other" means that a degree of difference between the templates of the both is smaller than a threshold. The more similar the two templates are, the degree of difference takes a smaller value, and the less similar the two templates are, the degree of difference takes a larger value. When the degree of difference between the both templates is 0, the both templates completely coincide with each other. Note that the "degree of difference" may be referred to as "score value".

Accordingly, having the template pair stored in the intermediate server 10-1, the UP template acquired in the mobile terminal 30-1 and the LP template stored in the transaction server 20-1 can be associated with each other. Therefore, in the disclosed technique, a transaction is enabled between the mobile terminal 30-1 and the transaction server 20-1 by performing personal authentication through the intermediate server 10-1 as described below.

### <Processing Sequence of Network System>

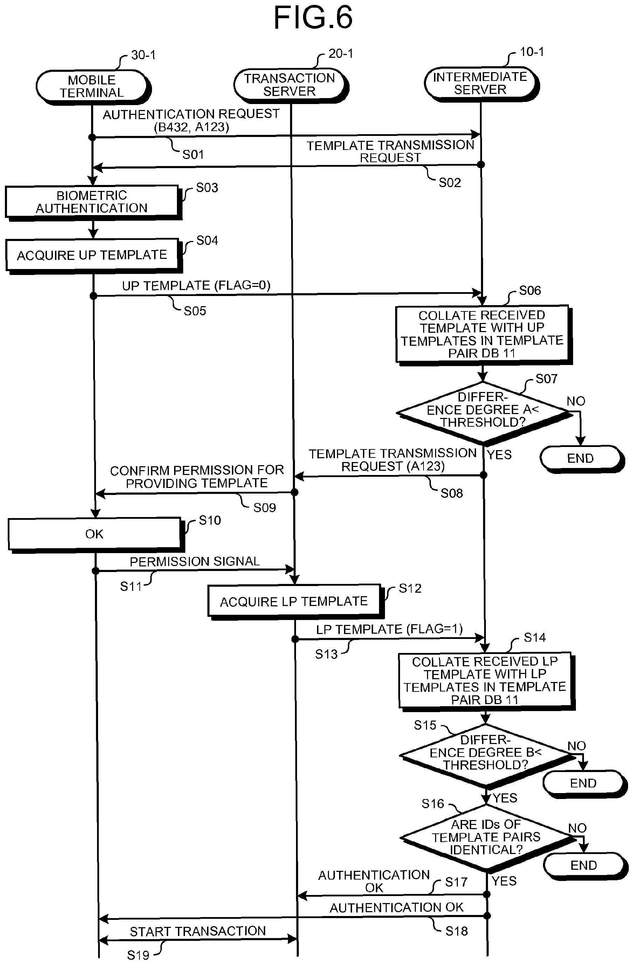

FIG. 6 is a diagram depicting one example of a processing sequence of a network system of the first embodiment.

First, when an input operation for making an authentication request is made to the mobile terminal 30-1 by a user of the mobile terminal 30-1, the authentication control unit 32 of the mobile terminal 30-1 outputs the authentication request to the communication unit 31 according to the input operation, and the communication unit 31 transmits the authentication request to the intermediate server 10-1 (step S01). The authentication control unit 32 includes the user ID=B432 and the template ID=A123 in the authentication request. The user ID=B432 and the template ID=A123 included in the authentication request are ones input to the mobile terminal 30-1 by the person "A" that is the user of the mobile terminal 30-1, and the person "A" is aware of them. For example, the user ID=B432 also serves as a login ID to the mobile terminal 30-1, and the template ID=A123 also serves as an account number of a transaction account for which account activity data is updated. That is, in the following, a case in which the transaction server 20-1 is a server that manages a bank account, and the user of the mobile terminal 30-1 makes a banking transaction with the transaction server 20-1 using the UP template as a collation template is explained as an example.

The communication unit 14 of the intermediate server 10-1 receives the authentication request from the mobile terminal 30-1, and outputs it to the template collating unit 12. The template collating unit 12 to which the authentication request is input outputs a template transmission request to the communication unit 14, and the communication unit 14 transmits the template transmission request to the mobile terminal 30-1 (step S02). Moreover, the template collating unit 12 to which the authentication request is input extracts the user ID=B432 and the template ID=A123 from the authentication request to store therein.

The communication unit 31 of the mobile terminal 30-1 receives the template transmission request from the intermediate server 10-1 and outputs it to the authentication control unit 32, and the authentication control unit 32 outputs the input template transmission request to the template collating unit 33. The template collating unit 33 collates the UP template input as the collation template and the UP template stored as the registration template in the template storage unit 35, to perform biometric authentication (step S03). The template collating unit 33 outputs, when the collation template coincides with the registration template, a control signal indicating an authentication result is "OK" to the template acquiring unit 34. On the other hand, when the collation template does not coincide with the registration template, the template collating unit 33 determines that the authentication is "NG". Note that the collation template coincides with the registration template means that the degree of difference between the collation template and the registration template is smaller than a threshold, and that the collation template does not coincide with the registration template means that the degree of difference between the collation template and the registration template is equal to or larger than the threshold.

The template acquiring unit 34 to which the control signal indicating that the authentication result is "OK" is input acquires the UP template (namely, the UP template of the user ID=B432) stored in the template storage unit 35, and adds flag=0 to the acquired UP template to output to the communication unit 31 (step S04). Flag=0 signifies that the template is a UP template, and flag=1 signifies that the template is an LP template. The communication unit 31 then transmits the UP template to which flag=0 is added, to the intermediate server 10-1 (step S05).

Note that when the authentication result is "OK", the template collating unit 33 may output the UP template input as a collation template to the template acquiring unit 34, and the template acquiring unit 34 may output the UP template input by the template collating unit 33 to the communication unit 31, thereby transmitting the UP template being the collation template to the intermediate server 10-1 by the communication unit 31.

The communication unit 14 of the intermediate server 10-1 receives the UP template (flag=0) from the mobile terminal 30-1, and outputs it to the template collating unit 12. The template collating unit 12 determines from flag=0 that the template received from the mobile terminal 30-1 is a UP template. The template collating unit 12 collates the UP template received from the mobile terminal 30-1 with multiple UP templates stored in the template pair DB 11 (step S06). The template collating unit 12 calculates the degree of difference between the UP template received from the mobile terminal 30-1 and each of the UP templates stored in the template pair DB 11, and sets the smallest degree of difference as difference degree A. Moreover, the template collating unit 12 identifies an ID of a template pair that includes the UP template having the smallest degree of difference from the UP template received from the mobile terminal 30-1, out of the UP templates stored in the template pair DB 11. In this example, because the UP template of the user ID=B432 and the UP template included in the template pair of ID=C897 are acquired from the same person, ID=C897 is identifies as the ID of the template pair that includes the UP template having the smallest degree of difference from the UP template of the user ID=B432.

Subsequently, the template collating unit 12 determines whether difference degree A is smaller than the threshold (step S07). When difference degree A is equal to or larger than the threshold (step S07: NO), the authentication result is "NG (authentication failed)" and the processing is ended, and the transaction between the mobile terminal 30-1 and the transaction server 20-1 is disabled. On the other hand, when difference degree A is smaller than the threshold (step S07: YES), the template collating unit 12 outputs a template transmission request including ID=A123 to the communication unit 14, and the communication unit 14 transmits the template transmission request including ID=A123 to the transaction server 20-1 (step S08). That difference degree A is smaller than the threshold means that both templates of a subject of collation coincide with each other. In this example, the UP template of the user ID=B432 and the UP template included in the template pair of ID=C897 are ones acquired from the same person, and therefore, the degree of difference between the UP template of the user ID=B432 and the UP template included in the template pair of ID=C897 is smaller than the threshold.

The communication unit 23 of the transaction server 20-1 receives a transmission request including ID=A123, and outputs it to the template acquiring unit 22. The template acquiring unit 22 to which the template transmission request including ID=A123 is input outputs, to the communication unit 23, a template-provision permission confirmation to confirm permission for providing an LP template from the transaction server 20-1 to the intermediate server 10-1, and the communication unit 23 transmits the template-provision permission confirmation to the mobile terminal 30-1 (step S09).

The communication unit 31 of the mobile terminal 30-1 receives the template-provision permission confirmation, and outputs it to the authentication control unit 32. The authentication control unit 32 confirms with a user whether to permit provision of an LP template by using a display on a touch panel included in the mobile terminal 30-1 and the like. When the user makes an operation of inputting "OK" to this confirmation to the mobile terminal 30-1 (step S10), the authentication control unit 32 outputs a permission signal to the communication unit 31, and the communication unit 31 transmits the permission signal to the transaction server 20-1 (step S11).

The communication unit 23 of the transaction server 20-1 receives the permission signal, and outputs it to the template acquiring unit 22. The template acquiring unit 22 to which the template transmission request including ID=A123 and the permission signal are input extracts ID=A123 from the template transmission request, acquires the LP template of ID=A123 from the LP template DB 21, and adds flag=1 to the acquired LP template to output to the communication unit 23 (step S12). The communication unit 23 transmits the LP template to which flag=1 is added to the intermediate server 10-1 (step S13).

The communication unit 14 of the intermediate server 10-1 receives the LP template (flag=1) from the transaction server 20-1, and outputs it to the template collating unit 12. The template collating unit 12 determines from flag=1 that the template received from the transaction server 20-1 is an LP template. The template collating unit 12 collates the LP template received from the transaction server 20-1 with multiple LP templates that are stored in the template pair DB 11 (step S14). The template collating unit 12 calculates a degree of difference between the LP template received from the transaction server 20-1 with each of the LP templates stored in the template pair DB 11, and sets the smallest degree of difference as difference degree B. Moreover, the template collating unit 12 identifies an ID of a template pair that includes an LP template having the smallest degree of difference from the LP template received for the transaction server 20-1, out of the LP templates stored in the template pair DB 11. In this example, because the LP template of ID=A123 and the LP template included in the template pair of ID=C897 are ones acquired from the same person, ID=C897 is identified as the ID of the template pair including an LP template having the smallest degree of difference from the UP template of ID=A123.

Subsequently, the template collating unit 12 determines whether difference degree B is smaller than the threshold (step S15). When difference degree B is equal to or larger than the threshold (step S15: NO), the authentication result is "NG (authentication failed)", and the processing is ended, and the transaction between the mobile terminal 30-1 and the transaction server 20-1 is disabled. On the other hand, when difference degree B is smaller than the threshold (step S15: YES), the template collating unit 12 determines whether the ID identified in the collation at step S06 and the ID identified in the collation at step S14 coincide with each other (step S16). That is, the template collating unit 12 determines whether the ID of the template pair including the UP template having the smallest degree of difference from the UP template received from the mobile terminal 30-1 and the ID of the template pair including the LP template having the smallest degree of difference from the LP template received from the transaction server 20-1 coincide with each other.

Because a template pair is uniquely identified by an ID, that an ID of a first template pair and an ID of a second template pair coincide with each other means that the first template pair and the second template pair are the same template pair. Furthermore, that an ID of a template pair that includes a specific UP template and an ID of a second template pair that includes a specific LP template coincide with each other means that the specific UP template and the specific LP template are included in the same template pair and are paired therein.

When the IDs of the template pairs do not coincide with each other at step S16 (step S16: NO), the authentication result is "NG (authentication failed)", and the processing is ended, and the transaction between the mobile terminal 30-1 and the transaction server 20-1 is disabled. On the other hand, when the IDs of the template pairs coincide with each other at step S16 (step S16: YES), the template collating unit 12 determines that the authentication result is "OK (authentication succeeded)", and outputs a control signal indicating that the authentication result is "OK" to the communication unit 23. The communication unit 23 transmits this control signal to the transaction server 20-1 and the mobile terminal 30-1 (steps S17, S18). That difference degree B is smaller than the threshold means that both templates of a subject of collation coincide with each other. In this example, because the LP template of ID=A123 and the LP template included in the template pair of ID=C897 are ones acquired from the same person, the degree of difference between the LP template of ID=A123 and the LP template included in the template pair of ID=C897 is smaller than the threshold.

The communication unit 23 of the transaction server 20-1 receives the control signal indicating that the authentication result is "OK" from the intermediate server 10-1, and outputs it to the transaction performing unit 24. Moreover, the communication unit 31 of the mobile terminal 30-1 receives the control signal indicating that the authentication result is "OK" from the intermediate server 10-1, and outputs it to the transaction performing unit 36. Thus, a transaction is started between the transaction performing unit 24 of the transaction server 20-1 and the transaction performing unit 36 of the mobile terminal 30-1 (step S19).

As described above, in the first embodiment, the intermediate server 10-1 has the template pair DB 11 in which template pairs pairing a UP template and an LP template that are acquired from the same person are stored for multiple persons. The mobile terminal 30-1 transmits a UP template that is acquirable at the mobile terminal 30-1 to the intermediate server 10-1. The transaction server 20-1 transmits an LP template stored in the LP template DB 21 of the transaction server 20-1 to the intermediate server 10-1. The intermediate server 10-1 collates the UP template transmitted from the mobile terminal 30-1 with each of the template pairs of multiple persons while comparing the LP template transmitted from the transaction server 20-1 with each of the template pair of the multiple servers. The intermediate server 10-1 performs personal authentication according to whether the template pair including the UP template that coincides with the UP template transmitted from the mobile terminal 30-1 and the template pair including the LP template that coincides with the LP template transmitted from the transaction server 20-1 coincide with each other.

As described, by performing personal authentication between the mobile terminal 30-1 and the transaction server 20-1 through the intermediate server 10-1 having the template pair DB 11, personal authentication is enabled even when noise components included in a registration template that is registered in advance in the transaction server 20-1 and noise components included in a collation template that is acquirable at the mobile terminal 30-1 differ from each other.

### [Second Embodiment]

### <Configuration of Network System>

Because a configuration of a network system of a second embodiment is the same as that of the first embodiment (FIG. 1), explanation thereof is omitted.

### <Configuration of Intermediate Server>

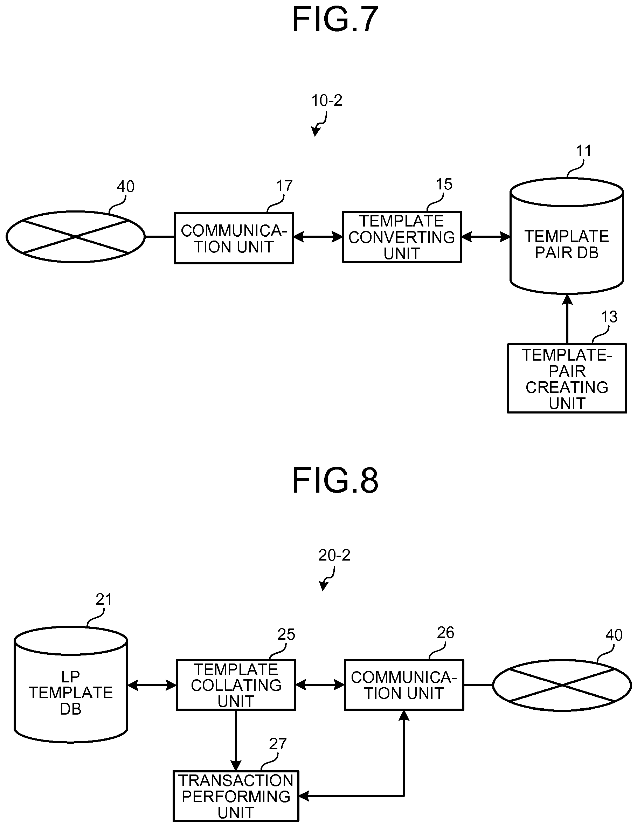

FIG. 7 is a functional block diagram depicting one example of an intermediate server of the second embodiment. An intermediate server 10-2 depicted in FIG. 7 corresponds to the intermediate server 10 depicted in FIG. 1. The intermediates server 10-2 includes a template converting unit 15, a communication unit 17 that is connected to the network 40, the template-pair creating unit 13, and the template pair DB 11.

The template converting unit 15 collates a collation template that is input from the communication unit 17 and a template pair that is stored in the template pair DB 11, and converts a UP template into an LP template. Moreover, the template converting unit 15 outputs the LP template obtained after conversion to the communication unit 17.

The communication unit 17 outputs an authentication request that is received from the mobile terminal 30 to the template converting unit 15. Furthermore, the communication unit 17 outputs a collation template that is received from the mobile terminal 30 to the template converting unit 15. Moreover, the communication unit 17 transmits an LP template that is input from the template converting unit 15 to the transaction server 20.

### <Configuration of Transaction Server>

FIG. 8 is a functional block diagram depicting one example of a transaction server of the second embodiment. A transaction server 20-2 depicted in FIG. 8 corresponds to the transaction server 20 depicted in FIG. 1. The transaction server 20-2 includes a template collating unit 25, a communication unit 26 that is connected to the network 40, a transaction performing unit 27, and the LP template DB 21.

The template collating unit 25 collates an LP template that is input from the communication unit 26 and an LP template that is stored in the LP template DB 21, and outputs an authentication result to the transaction performing unit 27 and the communication unit 26.

The communication unit 26 transmits the authentication result input from the template collating unit 25 to the mobile terminal 30. Moreover, the communication unit 26 receives a transaction command from the mobile terminal 30, and outputs it to the transaction performing unit 27. Furthermore, the communication unit 26 transmits the transaction command input from the transaction performing unit 27 to the mobile terminal 30.

The transaction performing unit 27 performs a transaction between itself and the mobile terminal 30 through the network 40 according to an authentication result input from the template collating unit 25. The transaction performing unit 27 receives various kinds of transaction commands input from the communication unit 26, and outputs various kinds of transaction commands to the communication unit 26, thereby performing a transaction between itself and the mobile terminal 30 through the network 40.

### <Configuration of Mobile Terminal>

Because a configuration of the mobile terminal of the second embodiment is the same as that of the first embodiment (FIG. 4), explanation thereof is omitted.

### <Processing Sequence of Network System>

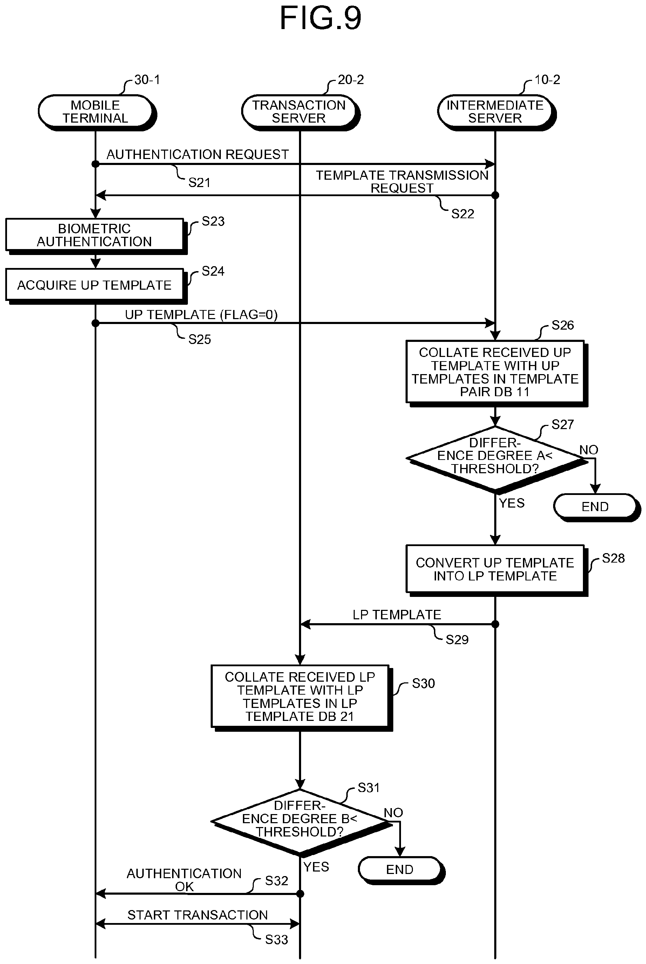

FIG. 9 is a diagram depicting one example of a processing sequence of the network system of the second embodiment.

First, when an input operation for making an authentication request is made to the mobile terminal 30-1 by a user of the mobile terminal 30-1, the authentication control unit 32 of the mobile terminal 30-1 outputs the authentication request to the communication unit 31 according to the input operation, and the communication unit 31 transmits the authentication request to the intermediate server 10-2 (step S21).

The communication unit 17 of the intermediate server 10-2 receives the authentication request from the mobile terminal 30-1, and outputs it to the template converting unit 15. The template converting unit 15 to which the authentication request is input outputs a template transmission request to the communication unit 17, and the communication unit 17 transmits the template transmission request to the mobile terminal 30-1 (step S22).

The communication unit 31 of the mobile terminal 30-1 receives the template transmission request from the intermediate server 10-2 and outputs it to the authentication control unit 32, and the authentication control unit 32 outputs the input template transmission request to the template collating unit 33. The template collating unit 33 collates a UP template input as a collation template and a UP template stored as a registration template in the template storage unit 35, to perform biometric authentication (step S23). The template collating unit 33 outputs, when the collation template coincides with the registration template, a control signal indicating an authentication result is "OK" to the template acquiring unit 34. On the other hand, when the collation template does not coincide with the registration template, the template collating unit 33 determines that the authentication is "NG".

The template acquiring unit 34 to which the control signal indicating that the authentication result is "OK" is input acquires, from the template storage unit 35, a UP template (namely, the UP template of the user ID=B432) stored in the template storage unit 35, and adds flag=0 to the acquired UP template to output to the communication unit 31 (step S24). The communication unit 31 then transmits the UP template to which flag=0 is added, to the intermediate server 10-2 (step S25).

Note that when the authentication result is "OK", the template collating unit 33 may output the UP template input as a collation template to the template acquiring unit 34, and the template acquiring unit 34 may output the UP template input by the template collating unit 33 to the communication unit 31, thereby transmitting the UP template being the collation template to the intermediate server 10-2 by the communication unit 31.

The communication unit 17 of the intermediate server 10-2 receives the UP template (flag=0) from the mobile terminal 30-1, and outputs it to the template converting unit 15. The template converting unit 15 determines from flag=0 that the template received from the mobile terminal 30-1 is a UP template. The template converting unit 15 collates the UP template received from the mobile terminal 30-1 with multiple UP templates stored in the template pair DB 11 (step S26). The template converting unit 15 calculates the degree of difference between the UP template received from the mobile terminal 30-1 and each of the UP templates stored in the template pair DB 11, and sets the smallest degree of difference as difference degree A. Moreover, the template converting unit 15 identifies an ID of a template pair that includes the UP template having the smallest degree of difference from the UP template received from the mobile terminal 30-1, out of the UP templates stored in the template pair DB 11. In this example, ID=C897 is identifies as the ID of the template pair that includes the UP template having the smallest degree of difference from the UP template of the user ID=B432.

Subsequently, the template converting unit 15 determines whether difference degree A is smaller than a threshold (step S27). When difference degree A is equal to or larger than the threshold (step S27: NO), the authentication result is "NG (authentication failed)" and the processing is ended, and the transaction between the mobile terminal 30-1 and the transaction server 10-2 is disabled. On the other hand, when difference degree A is smaller than the threshold (step S27: YES), the template converting unit 15 converts the UP template of the user ID=B432 into the LP template included in the template pair of ID=C897, and outputs the LP template obtained after conversion to the communication unit 17 (step S28). The communication unit 17 transmits the LP template input from the template converting unit 15 to the transaction server 20-2 (step S29). That difference degree A is smaller than the threshold means that both templates of a subject of collation coincide with each other. In this example, the degree of difference between the UP template of the user ID=B432 and the UP template included in the template pair of ID=C897 is smaller than the threshold.

The communication unit 26 of the transaction server 20-2 receives the LP template that is included in the template pair of ID=C897, and outputs it to the template collating unit 25. The template collating unit 25 collates the LP template received from the intermediate server 10-2 with each of the LP templates stored in the LP template DB 21 to calculate a degree of difference, and sets the smallest degree of difference as difference degree B (step S30). Moreover, the template collating unit 25 identifies a template ID of the LP template having the smallest degree of difference from the LP template received for the intermediate server 10-2, out of the LP templates stored in the LP template DB 21. Therefore, in this example, ID=A123 is identified as the ID of the LP template having the smallest degree of difference from the LP template included in the template pair of ID=C897.

Subsequently, the template collating unit 25 determines whether difference degree B is smaller than the threshold (step S31). When difference degree B is equal to or larger than the threshold (step S31: NO), the authentication result is "NG (authentication failed)", and the processing is ended, and the transaction between the mobile terminal 30-1 and the transaction server 20-2 is disabled. On the other hand, when difference degree B is smaller than the threshold (step S31: YES), the template collating unit 25 determines that the authentication result is "OK (authentication succeeded)", and outputs a control signal indicating that the authentication result is "OK" to the transaction performing unit 27 and the communication unit 26. The communication unit 26 transmits this control signal to the mobile terminal 30-1 (step S32). That difference degree B is smaller than the threshold means that both templates of a subject of collation coincide with each other. In this example, the degree of difference between the LP template included in the template pair of ID=C897 and the LP template of ID=A123 is smaller than the threshold.

The communication unit 31 of the mobile terminal 30-1 receives the control signal indicating that the authentication result is "OK" from the transaction server 20-2, and outputs it to the transaction performing unit 36. Thus, a transaction is started between the transaction performing unit 27 of the transaction server 20-2 and the transaction performing unit 36 of the mobile terminal 30-1 (step S33).

As described above, in the second embodiment, the intermediate server 10-2 has the template pair DB 11 in which template pairs pairing a UP template and an LP template that are acquired from the same person are stored for multiple persons. The mobile terminal 30-1 transmits a UP template that is acquirable at the mobile terminal 30-1 to the intermediate server 10-2. The intermediate server 10-2 converts the UP template transmitted from the mobile terminal 30-1 into an LP template by using a template pair stored in the template pair DB 11, and transmits the LP template obtained after conversion to the transaction server 20-2. The transaction server 20-2 performs personal authentication by collating the LP template transmitted from the intermediate server 10-2 with each of LP templates stored in the LP template DB 21 of multiple persons.

As described, by performing personal authentication between the mobile terminal 30-1 and the transaction server 20-2 through the intermediate server 10-2 having the template pair DB 11, personal authentication is enabled even when noise components included in a registration template that is registered in advance in the transaction server 20-2 and noise components included in a collation template that is acquirable at the mobile terminal 30-1 differ from each other.

### [Third Embodiment]

### <Configuration of Network System>

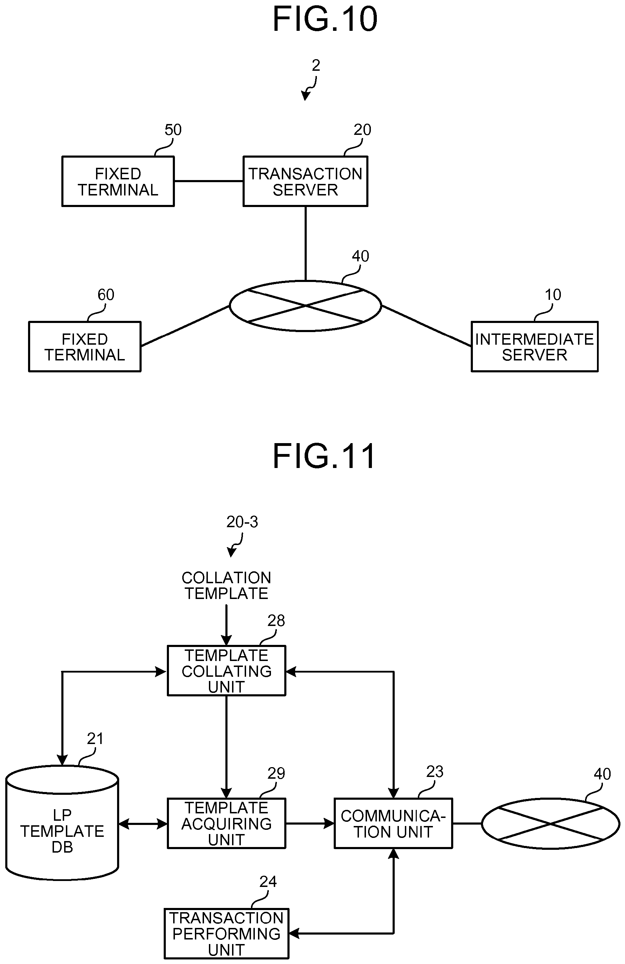

FIG. 10 is a diagram depicting one example of a configuration of a network system of a third embodiment. A network system 2 depicted in FIG. 10 includes the intermediates server 10, the transaction server 20, and fixed terminals 50, 60. The fixed terminal 50 is connected to the transaction server 20. The intermediate server 10, the transaction server 20, and the fixed terminal 60 are connected through the network 40.

### <Configuration of Intermediate Server>

Because a configuration of the intermediate server of the third embodiment is the same as that of the first embodiment (FIG. 2), explanation thereof is omitted.

### <Configuration of Transaction Server>

FIG. 11 is a functional block diagram depicting one example of the transaction server of the third embodiment. A transaction server 20-3 depicted in FIG. 11 corresponds to the transaction server 20 depicted in FIG. 10. The transaction server 20-3 includes a template collating unit 28, a template acquiring unit 29, the LP template DB 21, the communication unit 23, and the transaction performing unit 24.

To the template collating unit 28, an LP template that is acquired by the fixed terminal 50 is input as a collation template. The template collating unit 28 collates the LP template input from the fixed terminal 50 and the LP template that is stored in the LP template DB 21, and outputs an authentication result to the template acquiring unit 29.

The template acquiring unit 29 acquires an P template from the LP template DB 21 according to an authentication result input from the template collating unit 28, and outputs it to the communication unit 23.

### <Configuration of Fixed Terminal>

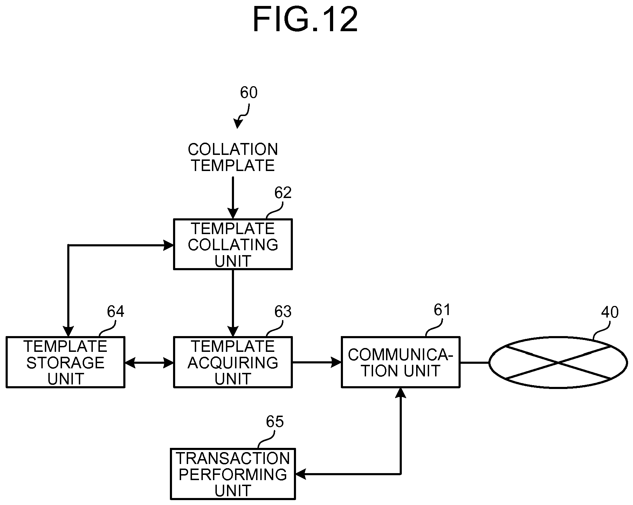

FIG. 12 is a functional block diagram depicting one example of a fixed terminal of the third embodiment. In FIG. 12, the fixed terminal 60 includes a communication unit 61 that is connected to the network 40, a template collating unit 62, a template acquiring unit 63, a template storage unit 64 and a transaction performing unit 65.

In the template storage unit 64, a UP template that is registered in advance by a user of the fixed terminal 60 is stored as a registration template. For example, in the template storage unit 64, a UP template of the user of the fixed terminal 60 is stored with the unique user ID "B432" added thereto. For example, the user of the fixed terminal 60 is the above person "A". That is, the user ID of the person "A" is "B432".

The template collating unit 62 collates a UP template that is input as a collation template and a UP template that is stored in the template storage unit 64 as a registration template, and outputs an authentication result to the template acquiring unit 63. The UP template input to the template collating unit 62 is one acquired from a vein pattern that is imaged by the unpolarization method by an imaging unit (not depicted) included in the fixed terminal 60 at the time of personal authentication. A collation-template creating unit (not depicted) included in the fixed terminal 60 creates a UP template as a collation template from a vein pattern that is imaged by the unpolarization method, and outputs it to the template collating unit 62.

The template acquiring unit 63 acquires a UP template from the template storage unit 64 according to an authentication result input from the template collating unit 62, to output to the communication unit 61.

The communication unit 61 transmits the UP template input from the template acquiring unit 63 to the intermediate server 10-1. Moreover, the communication unit 61 outputs a control signal received from the intermediate server 10-1 to the template acquiring unit 63. Furthermore, the communication unit 61 outputs an authentication result received from the intermediate server 10-1 to the transaction performing unit 65. Moreover, the communication unit 61 receives a transaction command from the transaction server 20-3, to output to the transaction performing unit 65. Furthermore, the communication unit 61 transmits a transaction command input from the transaction performing unit 65, to the transaction server 20-3.

The transaction performing unit 65 performs a transaction between itself and the transaction server 20-3 through the network 40 according to an authentication result input from the communication unit 61. The transaction performing unit 65 performs a transaction between itself and the transaction server 20-3 through the network 40 by receiving various kinds of transaction commands input from the communication unit 61, and by outputting various kinds of transaction commands to the communication unit 61.

### <Processing Sequence of Network System>

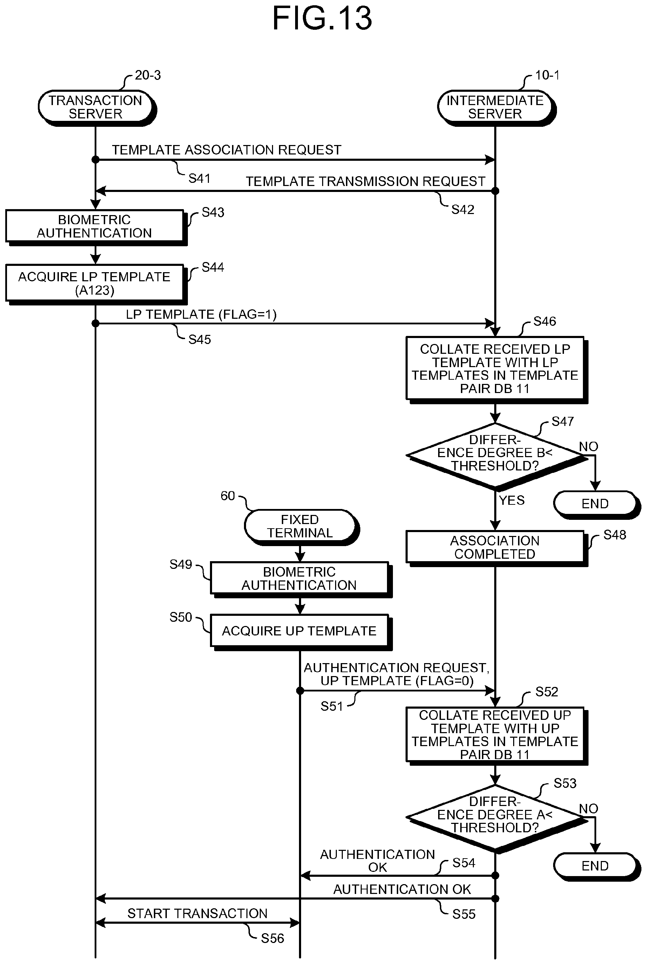

FIG. 13 is a diagram depicting one example of a processing sequence of the network system of the third embodiment.

First, the template collating unit 28 of the transaction server 20-3 outputs, when a collation template is input, a "template association request" to request association of an LP template that is stored in the LP template DB 21 and a template pair that is stored in the template pair DB 11 to the communication unit 23, and the communication unit 23 transmits the template association request to the intermediate server 10-1 (step S41).

The communication unit 14 of the intermediate server 10-1 receives the template association request from the transaction server 20-3, and outputs it to the template collating unit 12. The template collating unit 12 to which the template association request is input outputs a template transmission request to the communication unit 14, and the communication unit 14 transmits the template transmission request to the transaction server 20-3 (step S42) .

The communication unit 23 of the transaction server 20-3 receives the template transmission request from the intermediate server 10-1, and outputs it to the template collating unit 28. The template collating unit 28 collates the LP template that is input as a collation template with multiple templates that are stored as a registration template in the LP template DB 21, thereby performing biometric authentication (step S43). The template collating unit 28 outputs, when the collation template coincides with either one of the registration templates, a control signal indicating an authentication result is "OK" to the template acquiring unit 29. On the other hand, when the collation template does not coincide with any of the registration templates, the template collating unit 28 determines that the authentication is "NG". Note that the collation template coincides with either one of the registration templates means that the smallest degree of difference among degrees of difference between the collation template and the registration templates is smaller than a threshold. Moreover, that the collation template does not coincide with any of the registration templates means that any of the degrees of difference between the collation template and the registration templates is equal to or larger than the threshold.

The template acquiring unit 29 to which the control signal indicating that the authentication result is "OK" is input acquires an LP template (namely, the LP template of ID=A123) that coincides with the collation template from the LP template DB 21, and adds ID=A123 and flag=1 to the acquired LP template to output to the communication unit 23 (step S44). The communication unit 23 then transmits the LP template to which ID=A123 and flag=1 are added, to the intermediate server 10-1 (step S45).

Note that when the authentication result is "OK", the template collating unit 28 may output the LP template input as a collation template to the template acquiring unit 29, and the template acquiring unit 29 may output the LP template input by the template collating unit 28 to the communication unit 23, thereby transmitting the LP template being the collation template to the intermediate server 10-1 by the communication unit 23.

The communication unit 14 of the intermediate server 10-1 receives the LP template (flag=1) from the transaction server 20-3, and outputs it to the template collating unit 12. The template collating unit 12 determines from flag=1 that the template received from the transaction server 20-3 is an LP template. Moreover, the template collating unit 12 recognizes that the ID of the LP template received from the transaction server 20-3 is "A123". The template collating unit 12 collates the LP template received from the transaction server 20-3 with multiple LP templates stored in the template pair DB 11 (step S46). The template collating unit 12 calculates the degree of difference between the LP template received from the transaction server 20-3 and each of the LP templates stored in the template pair DB 11, and sets the smallest degree of difference as difference degree B. Moreover, the template collating unit 12 identifies an ID of a template pair that includes the LP template having the smallest degree of difference from the LP template received from the transaction server 20-3, out of the LP templates stored in the template pair DB 11. In this example, ID=C897 is identified as the ID of the template pair that includes the LP template having the smallest degree of difference from the LP template of the user ID=A123. Furthermore, the degree of difference between the LP template of ID=A123 and the LP template included in the template pair of ID=C897 is smaller than the threshold.

Subsequently, the template collating unit 28 determines whether difference degree B is smaller than the threshold (step S47). When difference degree B is equal to or larger than the threshold (step S47: NO), the authentication result is "NG (authentication failed)" and the processing is ended, and the transaction between the fixed terminal 60 and the transaction server 20-3 is disabled. On the other hand, when difference degree B is smaller than the threshold (step S47: YES), the template collating unit 28 associates ID=A123 and ID=C897 (step S48). Thus, the LP template of ID=A123 that is transmitted from the transaction server 20-3 and the template pair of ID=C897 that is stored in the template pair DB 11 are associated with each other.

When an input operation of making an authentication request to the fixed terminal 60 is made by the user of the fixed terminal 60 after association at step S48 is completed, the template collating unit 62 collates a UP template input as a collation template with a UP template that is stored as a registration template in the template storage unit 64, to perform biometric authentication (step S49). The template collating unit 62 outputs, when the collation template coincides with the registration template, a control signal indicating that an authentication result is "OK" to the template acquiring unit 63. On the other hand, when the collation template does not coincide with the registration template, the template collating unit 62 determines that the authentication is "NG".

The template acquiring unit 63 to which the control signal indicating that the authentication result is "OK" is input acquires the UP template (namely, the UP template of the user ID=B432) stored in the template storage unit 64, and adds flag=0 to the acquired UP template to output to the communication unit 61 (step S50). Furthermore, the template acquiring unit 63 outputs the authentication request to the communication unit 61. The communication unit 61 then transmits the UP template to which flag=0 is added, to the intermediate server 10-1 (step S51).

Note that when the authentication result is "OK", the template collating unit 62 may output the UP template input as a collation template to the template acquiring unit 63, and the template acquiring unit 63 may output the UP template input by the template collating unit 62 to the communication unit 61, thereby transmitting the UP template being the collation template to the intermediate server 10-1 by the communication unit 61.

The communication unit 14 of the intermediate server 10-1 receives the authentication request and the UP template (flag=0) from the fixed terminal 60, and outputs them to the template collating unit 12. The template collating unit 12 to which the authentication request is input determines from flag=0 that the template received from the fixed terminal 60 is a UP template. The template collating unit 12 collates the UP template received from the fixed terminal 60 with the UP template included in the template pair of ID=C897 associated at step S48 (step S52). The template collating unit 12 calculates the degree of difference between the UP template received from the fixed terminal 60 (namely, the UP template of the user ID=B432) and the UP template included in the template pair of ID=C897, and sets the calculated degree of difference as difference degree A. In this example, the degree of difference between the UP template of the user ID=B432 and the UP template included in the template pair of ID=C897 is smaller than the threshold.

Subsequently, the template collating unit 12 determines whether difference degree A is smaller than the threshold (step S53). When difference degree A is equal to or larger than the threshold (step S53: NO), the authentication result is "NG (authentication failed)" and the processing is ended, and the transaction between the fixed terminal 60 and the transaction server 20-3 is disabled. On the other hand, when difference degree A is smaller than the threshold (step S53: YES), the template collating unit 12 determines that the authentication is "OK (authentication succeeded)", and outputs a control signal indicating that the authentication result is "OK" to the communication unit 23. The communication unit 23 transmits this control signal to the transaction server 20-3 and the fixed terminal 60 (steps S54, S55).

The communication unit 23 of the transaction server 20-3 receives the control signal indicating that the authentication result is "OK" from the intermediate server 10-1, and outputs it to the transaction performing unit 24. Moreover, the communication unit 61 of the fixed terminal 60 receives the control signal indicating that the authentication result is "OK" from the intermediate server 10-1, and outputs it to the transaction performing unit 65. Thus, a transaction is started between the transaction performing unit 24 of the transaction server 20-3 and the transaction performing unit 65 of the fixed terminal 60 (step S56).

As described above, in the third embodiment, the intermediate server 10-1 has the template pair DB 11 in which template pairs pairing a UP template and an LP template that are acquired from the same person are stored for multiple persons. The transaction server 20-3 transmits an LP template stored in the LP template DB 21 of the transaction server 20-3 to the intermediate server 10-1. The intermediate server 10-1 associates the LP template transmitted from the transaction server 20-3 with a template pair stored in the template pair DB 11. The fixed terminal 60 transmits a UP template that is acquirable at the fixed terminal 60 to the intermediate server 10-1. The intermediate server 10-1 performs personal authentication by collating the template pair that is associated with the LP template transmitted from the transaction server 20-3 with the UP template that is transmitted from the fixed terminal 60.

As described, by performing personal authentication between the fixed terminal 60 and the transaction server 20-3 through the intermediate server 10-1 having the template pair DB 11, personal authentication is enabled even when noise components included in a registration template that is registered in advance in the transaction server 20-3 and noise components included in a collation template that is acquirable at the fixed terminal 60 differ from each other.

### [Fourth Embodiment]

### <Configuration of Network System>

Because a configuration of a network system of a fourth embodiment is the same as that of the first embodiment (FIG. 1), explanation thereof is omitted.

### <Configuration of Mobile Terminal>

Because a configuration of a mobile terminal of the fourth embodiment is the same as that of the first embodiment (FIG. 4), explanation thereof is omitted.

### <Configuration of Intermediate Server>

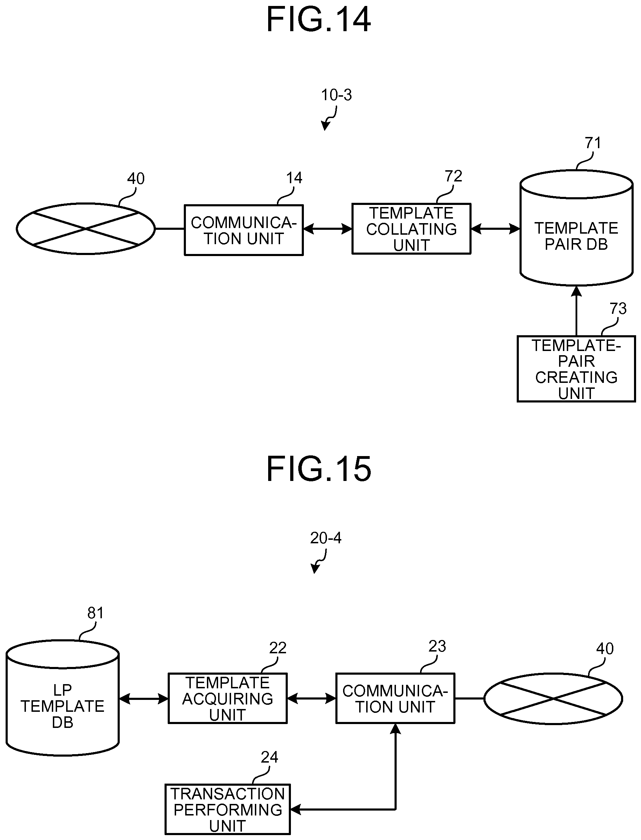

FIG. 14 is a functional block diagram depicting one example of an intermediate server of the fourth embodiment. An intermediate server 10-3 depicted in FIG. 14 corresponds to the intermediate server 10 depicted in FIG. 1. The intermediate server 10-3 includes a template pair DB 71, a template collating unit 72, a template-pair creating unit 73, and the communication unit 14 that is connected to the network 40.

In the template pair DB 71, an LP template and a UP template that are acquired at the same time from the same person are paired, and stored as a template pair. Note that, as explained below, it differs from the template pair DB 11 of the first embodiment in a point that an LP template and a UP template stored in the template pair DB 71 are formed with hash values.

That is, the template-pair creating unit 73 uses, for example, the technique disclosed in the specification of Japanese Patent No. 4974543, and thereby acquires a vein pattern by the linear polarization method and a vein pattern by the unpolarization method by a single time of imaging from the same person, and creates an LP template and a UP template from the vein patterns that are acquired at a time from the same person. At this time, the template-pair creating unit 73 hashes characteristics that are extracted from the vein pattern acquired by the linear polarization method using a predetermined hash function, to form an LP template with a hash value obtained after hashing. Moreover, the template-pair creating unit 73 hashes characteristics that are extracted from the vein pattern acquired by the unpolarization method using a predetermined hash function, to form a UP template with a hash value obtained after hashing. Thus, the LP template and the UP template are to be hash values of, for example, 2048 bits expressed with '0' and '1'. The template-pair creating unit 13 pairs an LP template and a UP template that are created from vein patterns acquired at a time from the same person to create a template pair of each person, and outputs the created template pair to the template pair DB 71.

The template collating unit 72 collates a collation template that is input from the communication unit 14 and a template pair that is stored in the template pair DB 71, and outputs an authentication result to the communication unit 14. Note that the template collating unit 72 differs from the template collating unit 12 of the first embodiment in a point that hash values are used in collation. Therefore, the template collating unit 72 hashes a collation template that is received from the mobile terminal 30-1 by using a predetermined hash function.

### <Configuration of Transaction Server>

FIG. 15 is a functional block diagram depicting one example of a transaction server of the fourth embodiment. A transaction server 20-4 depicted in FIG. 15 corresponds to the transaction server 20 depicted in FIG. 1. The transaction server 20-4 includes an LP template DB 81, the template acquiring unit 22, the communication unit 23 that is connected to the network 40, and the transaction performing unit 24.

In the LP template DB 81, multiple LP templates that are respectively acquired from multiple persons are stored as a registration template. Note that it differs from the LP template DB 21 of the first embodiment in a point that the LP templates stored in the LP template DB 81 are formed with hash values. That is, in the transaction server 20-4, characteristics extracted from a vein pattern that is acquired by the linear polarization method are hashed by using a predetermined hash function, and an LP template is formed with a hash value obtained after hashing.

### <Processing Sequence of Network System>

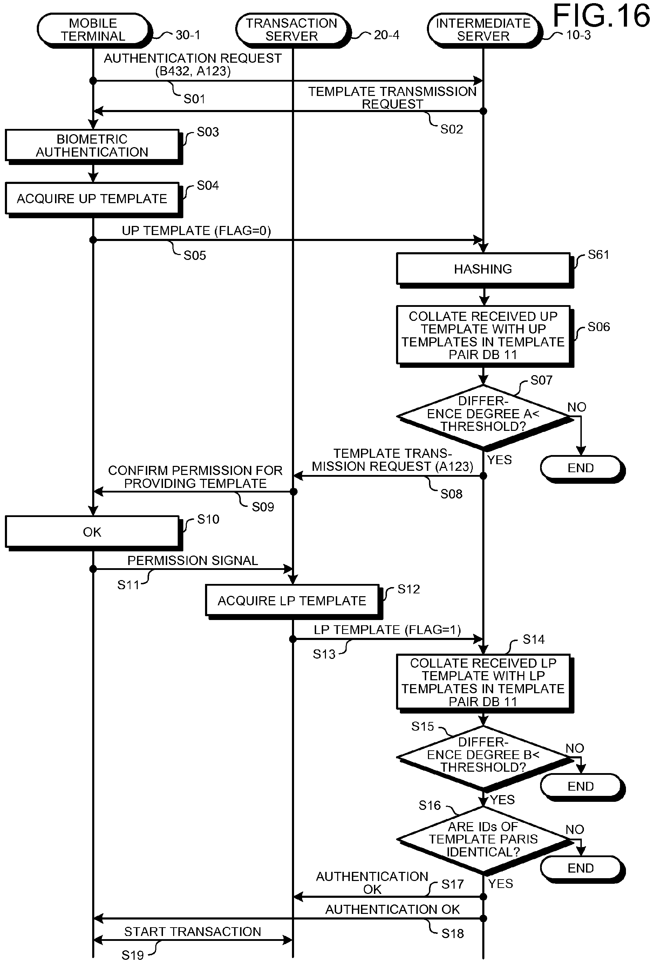

FIG. 16 is a diagram depicting one example of a processing sequence of the network system of the fourth embodiment. In FIG. 16, processing other than step S61 is the same as that of the first embodiment (FIG. 6). However, in the fourth embodiment, collation at step S06 and step S14 are performed as collation of hash values, and the LP template acquired at step S12 is formed with a hash value. Moreover, in the fourth embodiment, a degree of difference between templates is one obtained by comparing bits at the same position in the templates in bit sequences of hash values that are collated between templates, and by counting the number of bits that differ in value between templates.

The communication unit 31 of the mobile terminal 30-1 transmits the UP template to which flag=0 is added to the intermediate server 10-3 (step S05).

The communication unit 14 of the intermediate server 10-3 receives the UP template (flag=0) from the mobile terminal 30-1, and outputs it to the template collating unit 72. The template collating unit 72 hashes the UP template received from the mobile terminal 30-1 by using a predetermined hash function (step S61).

Moreover, the template collating unit 72 determines from flag=0 that the template received from the mobile terminal 30-1 is a UP template. The template collating unit 72 collates the hash value obtained after hashing at step S61 with the UP templates stored in the template pair DB 11 (step S06).

### [Fifth Embodiment]

### <Configuration of Network System>

Because a configuration of a network system of a fifth embodiment is the same as that of the first embodiment (FIG. 1), explanation thereof is omitted.

### <Configuration of Mobile Terminal>

Because a configuration of the mobile terminal of the fifth embodiment is the same as that of the first embodiment (FIG. 4), explanation thereof is omitted.

### <Configuration of Intermediate Server>

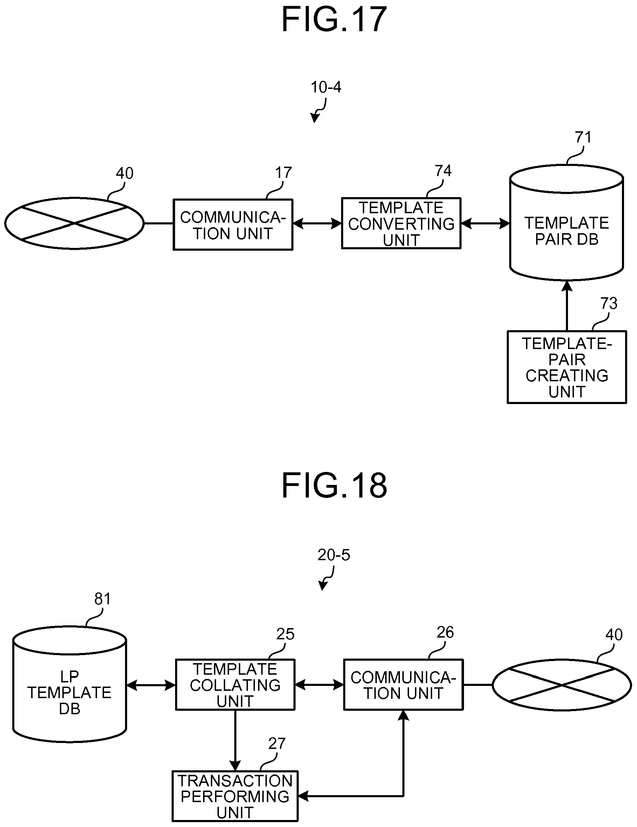

FIG. 17 is a functional block diagram depicting one example of an intermediate server of a fifth embodiment. An intermediate server 10-4 depicted in FIG. 17 corresponds to the intermediate server 10 depicted in FIG. 1. The intermediate server 10-4 includes a template converting unit 74, the communication unit 17 that is connected to the network 40, the template-pair creating unit 73, and the template pair DB 71.

The template converting unit 74 collates a collation template that is input from the communication unit 17 with a template pair that is stored in the template pair DB 71, and converts a UP template into an LP template. Furthermore, the template converting unit 74 outputs the LP template obtained after conversion to the communication unit 17. Note that the template converting unit 74 differs from the template converting unit 15 of the second embodiment in a point that collation is performed using hash values. Therefore, the template converting unit 74 hashes the collation template received from the mobile terminal 30-1 by using a predetermined hash function.

### <Configuration of Transaction Server>

FIG. 18 is a functional block diagram depicting one example of the transaction server of the fifth embodiment. A transaction server 20-5 depicted in FIG. 18 corresponds to the transaction server 20 depicted in FIG. 1. The transaction server 20-5 includes the template collating unit 25, the communication unit 26 that is connected to the network 40, the transaction performing unit 27, and an LP template DB 81. That is, the transaction server 20-5 differs from the transaction server 20-2 of the second embodiment in a point that the LB template DB 81 is included in place of the LB template DB 21.

### <Processing Sequence of Network System>

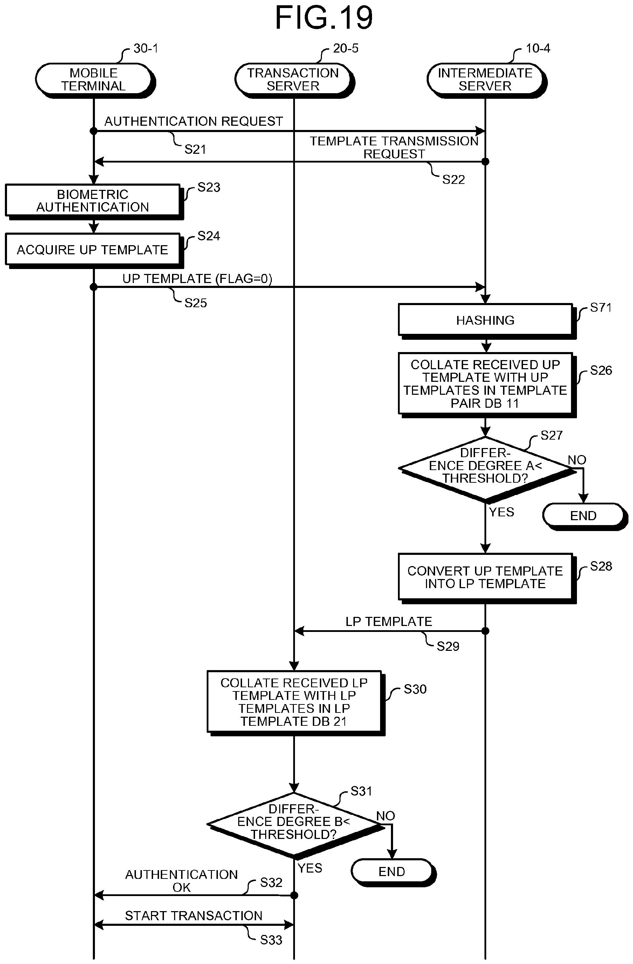

FIG. 19 is a diagram depicting one example of a processing sequence of the network system of the fifth embodiment. In FIG. 19, processing other than step S71 is the same as that of the second embodiment (FIG. 9). However, in the fifth embodiment, collation at step S26 and step S30 is performed as collation of hash values, and the LP template acquired at step S28 is formed with a hash value. Moreover, in the fifth embodiment, a degree of difference between templates is one obtained by comparing bits at the same position in the templates in bit sequences of hash values that are collated between templates, and by counting the number of bits that differ in value between templates.

The communication unit 31 of the mobile terminal 30-1 transmits the UP template to which flag=0 is added to the intermediate server 10-4 (step S25).

The communication unit 17 of the intermediate server 10-4 receives the UP template (flag=0) from the mobile terminal 30-1, and outputs it to the template converting unit 74. The template converting unit 74 hashes the UP template received from the mobile terminal 30-1 by using a predetermined hash function (step S71).

Moreover, the template converting unit 74 determines from flag=0 that the template received from the mobile terminal 30-1 is a UP template. The template converting unit 74 collates the hash value obtained after hashing at step S71 with the UP templates stored in the template pair DB 71 (step S26).

### [Sixth Embodiment]

### <Configuration of Network System>

Because a configuration of a network system of a sixth embodiment is the same as that of the third embodiment (FIG. 10), explanation thereof is omitted.

### <Configuration of Fixed Terminal>

Because a configuration of the fixed terminal of the sixth embodiment is the same as that of the third embodiment (FIG. 12), explanation thereof is omitted.

### <Configuration of Intermediate Server>

Because a configuration of the intermediate server of the sixth embodiment is the same as that of the fourth embodiment (FIG. 14), explanation thereof is omitted.

### <Configuration of Transaction Server>

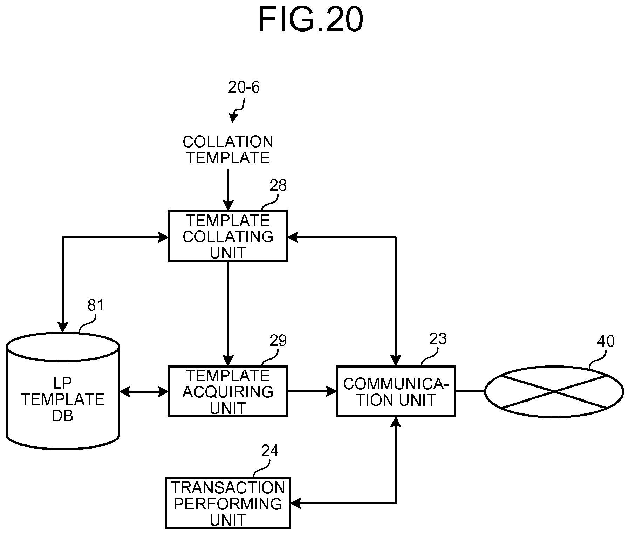

FIG. 20 is a functional block diagram depicting one example of a transaction server of the sixth embodiment. A transaction server 20-6 depicted in FIG. 20 corresponds to the transaction server 20 depicted in FIG. 10. The transaction server 20-6 includes the template collating unit 28, the template acquiring unit 29, the LP template DB 81, the communication unit 23, and the transaction performing unit 24. That is, the transaction server 20-6 differs from the transaction server 20-3 of the third embodiment in a point that the LB template DB 81 is included in place of the LB template DB 21.

### <Processing Sequence of Network System>

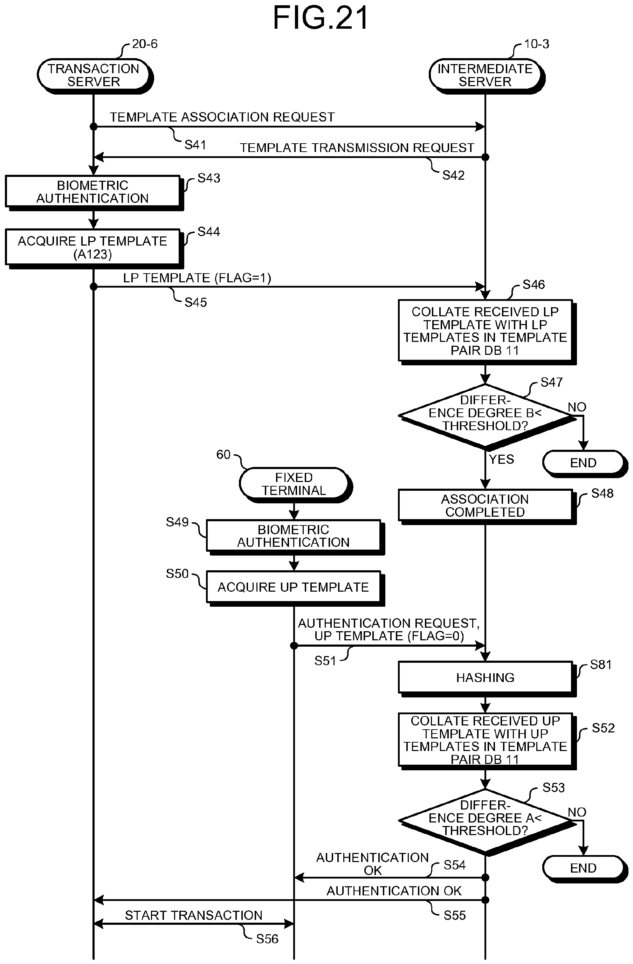

FIG. 21 is a diagram depicting one example of a processing sequence of the network system of the sixth embodiment. In FIG. 21, processing other than step S81 is the same as that of the third embodiment (FIG. 13). However, in the sixth embodiment, collation at step S46 and step S52 is performed as collation of hash values, and the LP template acquired at step S44 is formed with a hash value. Moreover, in the sixth embodiment, a degree of difference between templates is one obtained by comparing bits at the same position in the templates in bit sequences of hash values that are collated between templates, and by counting the number of bits that differ in value between templates.

The communication unit 61 of the fixed terminal 60 transmits an authentication request and the UP template to which flag=0 is added, to the intermediate server 10-3 (step S51).

The communication unit 14 of the intermediate server 10-3 receives the authentication request and the UP template (flag=0) from the fixed terminal 60, and outputs them to the template collating unit 72. The template collating unit 72 to which the authentication request is input hashes the UP template received from the fixed terminal 60 by using a predetermined hash function (step S81).

Moreover, the template collating unit 72 determines from flag=0 that the template received from the fixed terminal 60 is a UP template. The template collating unit 72 collates the hash value obtained after hashing at step S81 with the UP template that is included in the template pair of ID=C897 associated at step S48 (step S52).

As described above, in the fourth to the sixth embodiments, a UP template and an LP template in a template pair, and a UP template and an LP template being a subject of collation are formed with hash values. That is, in the fourth to the sixth embodiments, collation of templates is performed as collation of hash values. Therefore, collation in the fourth to the sixth embodiments can be performed by bit pattern matching, and therefore, time required for collation in personal authentication can be shortened in the fourth to the sixth embodiments, compared to the first to the third embodiments.

### [Other Embodiments]

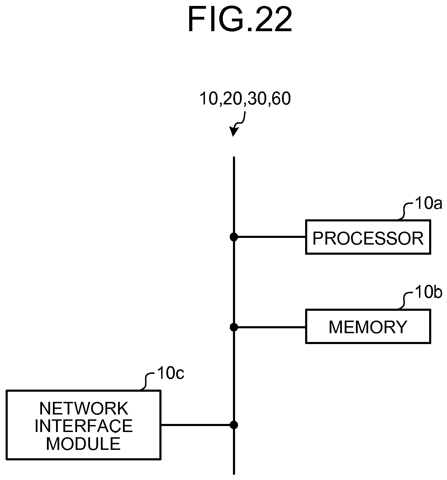

[1] The intermediate server 10, the transaction server 20, the mobile terminal 30, and the fixed terminal 60 can be implemented, for example by a hardware configuration as follows. FIG. 22 is a diagram depicting a hardware configuration example of the intermediate server, the transaction server, the mobile terminal, and the fixed terminal. As depicted in FIG. 22, the intermediate server 10, the transaction server 20, the mobile terminal 30, and the fixed terminal 60 include a processor 10a, a memory 10b, and a network interface module 10c as hardware components. As an example of the processor 10a, a CPU (central processing unit), a DSP (digital signal processor), a FPGA (field programmable gate array), and the like can be named. Moreover, the intermediates server 10, the transaction server 20, the mobile terminal 30, and the fixed terminal 60 may have an LSI (large scale integrated circuit) that includes the processor 10a and peripheral circuits. As an example of the memory 10b, a RAM (random access memory) such as an SDRAM (synchronous dynamic random access memory), a ROM (read only memory), a flash memory, and the like can be named. The communication units 14, 17, 23, 26, 31, and 61 are implemented by the network interface module 10c. The template collating units 12, 25, 28, 33, and 62, the template-pair creating unit 13, the template converting unit 15, the template acquiring units 22, 29, 34, and 63, the transaction performing unit 24, 27, 36, and 65, and the authentication control unit 32 are implemented by the processor 10a. The template pair DB 11, the LP template DB 21, and the template storage units 35 and 64 are implemented by the memory 10b.
   Furthermore, respective processing in the above explanation of the intermediate server 10, the transaction server 20, the mobile terminal 30, and the fixed terminal 60 may be implemented by executing a program that corresponds to each processing by the processor 10a. For example, a program that corresponds to each processing in the above explanation may be stored in a storage unit such as the memory 10b and an HDD (hard disk drive), and the program may be read from the storage unit to be executed by the processor 10a.
[2] In the above explanation, the LP template and the UP template are named as an example of templates including noise components different from each other. However, a polarization method of a template is not limited to the linear polarization method and the unpolarization method. In the disclosed technique, it is only necessary that the polarization method of a template that is registered in advance in the transaction server 20 (that is, a registration template) and the polarization method of a collation template that is acquirable at the mobile terminal 30 and the fixed terminal 60 at which personal authentication with respect to the transaction server 20 is desired to be performed.
[3] Names as "intermediate server" and "transaction server" that are used in the above explanation are one example, and names of servers are not limited to "intermediate server" and "transaction server".
[4] The biometric authentication to which the disclosed technique can be applied is not limited to noncontact vein pattern authentication using a palm vein pattern. The disclosed technique is applicable to various kinds of biometric authentication in which a template is acquired from an image of a living body.
[5] In the first embodiment, the template collating unit 12 determines, when difference degree A, which is the smallest among degrees of difference among UP templates, is smaller than a threshold, and when difference degree B, which is the smallest among degrees of difference among LP templates, is smaller than a threshold, whether an ID of a template pair that includes the UP template having difference degree A coincides with an ID of a template pair that includes the LP template having difference degree B, and determines that the authentication is "OK" when those IDs coincide with each other, determines that the authentication is "NG" when those IDs do not coincide with each other. However, the template collating unit 12 may perform authentication as in <authentication example 1> or <authentication example 2> below when more than one template pair for which the degree of difference is smaller than the threshold in collation of a UP template received from the mobile terminal 10-1 with UP templates of the template pair DB is present, and more than one template pair for which the degree of difference is smaller than the threshold in collation of an LP template received from the transaction server 20-1 with LP templates in the template pair DB 11 is present.

### <Authentication Example 1>

The template collating unit 12 determines that the authentication is "OK" when only one kind of ID is present that is identical among template pairs having a degree of difference smaller than a threshold in collation among UP templates and template pairs having a degree of difference smaller than the threshold in collation among LP templates, and determines that the authentication is "NG" when more than one kind of identical ID is present.

### <Authentication Example 2>

The template collating unit 12 determines that the authentication is "OK" when at least one kind of ID is present that is identical among template pairs having a degree of difference smaller than a threshold in collation among UP templates and template pairs having a degree of difference smaller than the threshold in collation among LP templates, and determines that the authentication is "NG" when an identical ID is not present. In other words, unlike authentication example 1, the template collating unit 12 determines that the authentication is "OK" even when two or more kinds of identical IDs are present in authentication example 2.

[6] In the first embodiment, a case in which the transaction server 20 is the only server is explained as an example. However, personal authentication may be performed between the mobile terminal 30 and each of multiple transaction servers 20, through the intermediate server 10.

### Explanation of Reference

1, 2 Network system
10, 10-1, 10-2, 10-4 Intermediate server
20, 20-1, 20-2, 20-3, 20-4, 20-5, 20-6 Transaction server
30, 30-1 Mobile terminal
50, 60 Fixed terminal
11, 71 Template pair DB
13, 73 Template-pair creating unit
14, 17, 23, 26, 31, 61 Communication unit
12, 25, 28, 33, 62, 72 Template collating unit
15, 74 Template converting unit
21, 81 LP template DB
22, 29, 34, 63 Template acquiring unit
24, 27, 36, 65 Transaction performing unit
32 Authentication control unit
35, 64 Template storage unit

## Claims

1. A server comprising:
a database that stores template pairs for a plurality of persons, the template pairs each pairing a first template and a second template that are acquired by using a first polarization method and a second polarization method different from each other, respectively, from a same person; and
a collating unit that collates a third template by the first polarization method, the third template being transmitted from a terminal device, with the template pairs for the persons, collates a fourth template by the second polarization method, the fourth template being transmitted from another server, with the template pairs for the persons, and performs personal authentication according to whether a first template pair and a second template pair are an identical template pair, the first template pair including the first template that coincides with the third template, and the second template pair including the second template that coincides with the fourth template.

2. The server according to claim 1, further comprising a creating unit that acquires the first template and the second template from the same person at a time by a single time of imaging, and creates the template pair by pairing the first template and the second template acquired from the same person at a time.

3. The server according to claim 1, wherein
out of the fist polarization method and the second polarization method, one is a unpolarization method, and the other is a linear polarization method.

4. The server according to claim 1, wherein
the collating unit determines an authentication result of the personal authentication as successful when the first template pair that includes the first template, a degree of difference from the third template of which is smallest and is smaller than a threshold and the second template pair that includes the second template, a degree of difference from the fourth template of which is smallest and is smaller than a threshold are an identical template pair.

5. The server according to claim 1, wherein
if a plurality of the first template pairs each of which includes the first template, a degree of difference from the third template of which is smaller than a threshold, and a plurality of the second template pairs each of which includes the second template, a degree of difference from the fourth template of which is smaller than a threshold are present, the collating unit determines an authentication result of the personal authentication as successful when only one kind of identical template pair is present among the first template pairs and the second template pairs.

6. The server according to claim 1, wherein
if a plurality of the first template pairs each of which includes the first template, a degree of difference from the third template of which is smaller than a threshold, and a plurality of the second template pairs each of which includes the second template, a degree of difference from the fourth template of which is smaller than a threshold are present, the collating unit determines an authentication result of the personal authentication as successful when more than one kind of identical template pair is present among the first template pairs and the second template pairs.

7. The server according to claim 1, wherein
the first template and the second template in the template pair, and the third template and the fourth template that are subject of collation by the collating unit are formed with hash values.

8. A network system comprising:
a first server;
a second server; and
a terminal device, wherein
the first server stores template pairs for a plurality of persons, the template pairs each pairing a first template and a second template that are acquired by using a first polarization method and a second polarization method different from each other, respectively, from a same person,
the terminal device transmits a third template by the first polarization method to the first server,
the second server transmits a fourth template by the second polarization method to the first server, and
the first server collates the third template with the template pairs for the persons, collates the fourth template with the template pairs for the persons, and performs personal authentication according to whether a first template pair and a second template pair are an identical template pair, the first template pair including the first template that coincides with the third template, and the second template pair including the second template that coincides with the fourth template.

9. A personal authentication method, comprising:
storing template pairs for a plurality of persons, the template pairs each pairing a first template and a second template that are acquired by using a first polarization method and a second polarization method different from each other, respectively, from a same person;
collating a third template that is acquired at a first device by the first polarization method with the template pairs for the persons;
collating a fourth template that is acquired at a second device different from the first device by the second polarization method with the template pairs for the persons; and
performing personal authentication according to whether a first template pair and a second template pair are an identical template pair, the first template pair including the first template that coincides with the third template, and the second template pair including the second template that coincides with the fourth template.
